# EUROPEAN PATENT APPLICATION

(11) **EP 4 744 649 A1**
(43) Date of publication of application: **20.05.2026**
(21) Application number: 24860519.8
(22) Date of filing: 23.07.2024
(51) Int. Cl.: A61K 9/00, A61K 47/34, A61K 31/00, A61K 9/14, A61P 25/00

(54) **SUSTAINED-RELEASE INJECTABLE COMPOSITION FOR TREATING OR PREVENTING NEUROLOGICAL DISORDERS, AND METHOD FOR PREPARING SAME**

(30) Priority: 25.08.2023 KR 20230111792; 12.03.2024 KR 20240034444
(71) Applicant: Inventage Lab Inc., Gyeonggi-do 13403 (KR); Yuhan Care Co., Ltd., Seoul 07789 (KR)
(72) Inventor: KIM, Ju Hee, Seongnam-si, Gyeonggi-do 13494 (KR); SEO, Young Dai, Seongnam-si, Gyeonggi-do 13494 (KR); PARK, Hyun Je, Gunpo-si, Gyeonggi-do 15889 (KR); CHA, Joo Young, Seongnam-si, Gyeonggi-do 13494 (KR); SONG, Aeri, Hwaseong-si, Gyeonggi-do 18441 (KR)
(74) Representative: BCKIP Part mbB
(86) International application number: PCT/KR2024/095928
(87) International publication number: WO 2025/048603

(57) **Abstract**

The present invention relates to: a sustained-release injectable composition for treating or preventing neurological disorders; and a method for preparing same. The sustained-release injectable composition uses microparticles containing cannabidiol and continuously releases the cannabidiol for at least one month, and thus can be effective in treating or preventing neurological disorders. In addition, the sustained-release injectable composition includes cannabidiol, which is non-toxic and can be used continuously for a long time due to the absence of side effects from long-term use, and thus can exhibit a therapeutic or prophylactic effect on neurological disorder patients for at least one month through a single injection. Therefore, the sustained-release injectable composition can greatly improve the convenience of administration.

## Description

### Technical Field

The present invention relates to a sustained-release injectable composition for treating or preventing a neurological disease and a method for preparing the same.

### Background Art

Epilepsy is a disease that causes an intermittent disorder of the nervous system due to a paroxysmal discharge of cerebral neurons resulting from organic lesions or functional disorders, and shows symptoms such as neurological symptoms, loss of consciousness, convulsions, and sensory disturbances. It is the third most common neurological disease after Alzheimer's disease and stroke, and approximately 0.5% to 2% of the world's population suffers from epilepsy.

Furthermore, approximately 45 new cases per 100,000 people occur worldwide each year, and it is estimated that there are approximately 300,000 to 400,000 epilepsy patients in Korea. Looking at the age distribution of epilepsy patients, 70% of all epilepsy cases are known to begin in childhood and adolescence, with a particularly high incidence in infancy. In addition, the incidence and prevalence rates are highest within the first year of life, then decrease rapidly, and then increase rapidly again in the elderly over 60 years of age, showing a U-shaped pattern, and the lifetime prevalence of experiencing seizures is 10 to 15%.

Epilepsy is a chronic disease characterized by recurrent epileptic seizures. Its causes are diverse, and the precise cause remains unknown. However, as recent advancements in neuroimaging technology have made it possible to observe subtle pathological changes in the brain that were previously unobservable, the understanding of the causes of epilepsy is gradually expanding. Some epidemiological studies have reported that more than one-third of patients have a history of pathological changes in the brain or brain damage, and it is known that the main causes are stroke, congenital malformation, head trauma, encephalitis, brain tumor, degenerative encephalopathy, heredity, prematurity, damage before and after birth, and the like.

Meanwhile, therapies for epilepsy can be broadly categorized into drug therapy and non-drug therapy, such as surgery, the ketogenic diet, or vagus nerve stimulation. However, non-drug therapy is performed only on patients who show drug resistance, and thus drug therapy has been used as the primary method for treating epilepsy. Conventional drugs include phenytoin (Dilantin), valproate (Orfil, Depakine, Depakote), carbamazepine (Tegretol), phenobarbital (Luminal), and ethosuximide (Zarontin). Recently, topiramate (Topamax), lamotrigine (Lamictal), vigabatrin (Sabril), oxcarbazepine (Trileptal), and the like have been developed and commercialized. However, these drugs are known to have side effects such as rash, blood disorders, osteoporosis, hyponatremia, headache, behavioral changes, and immune responses.

Several studies reported that cannabidiol (CBD), a major component of cannabis, has minimal psychoactive effects and exhibits anticonvulsant efficacy by suppressing neuronal hyperexcitability. Based on its efficacy and safety, Epidyolex^{®}, an oral liquid containing cannabidiol, was approved for marketing in 2018 and is currently being used for the treatment of rare pediatric epilepsy. However, because of the liquid form, it is difficult to administer the correct dosage to children, and there is a risk of relapse due to rapid loss of blood drug concentration.

Therefore, there is a need to develop a product as a therapeutic agent for the treatment or alleviation of epilepsy that has no side effects and may have an enhanced effect of treating or preventing epilepsy through long-term sustained drug release by a single injection.

### [Prior Art Documents]

### [Patent Documents]

(Patent Document 1) KR 10-2020-0105198 A1

### DISCLOSURE

### Technical Problem

An object of the present invention is to provide a sustained-release injectable composition for treating or preventing a neurological disease and a method for preparing the same.

Another object of the present invention is to provide a sustained-release injectable composition that may exhibit an effect of treating or preventing a neurological disease by releasing cannabidiol in a sustained manner for one month or more using microparticles containing cannabidiol.

Still another object of the present invention is to provide a method for preparing a sustained-release injectable composition containing cannabidiol, which is non-toxic, has no side effects resulting from long-term use, and thus may be used in a sustained manner for a long period of time, in which the sustained-release injectable composition may exhibit a therapeutic or preventive effect on a neurological disease for one month or more by a single injection, thereby significantly improving the convenience of administration.

### Technical Solution

To achieve the above objects, the present invention relates to a sustained-release injectable composition for treating or preventing a neurological disease, including microparticles that release cannabidiol (CBD) in a sustained manner for one month or more, wherein the microparticles include cannabidiol and a biodegradable polymer.

In addition, the microparticles may include cannabidiol and the biodegradable polymer at a weight ratio of 1:1 to 1:10.

In addition, the biodegradable polymer may be selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof.

The polylactide-co-glycolide may have a monomer ratio of lactide: glycolide of 40:60 to 90:10.

In addition, the neurological disease may be epilepsy.

In addition, the microparticles may release 35% to 80% of the total weight of CBD, as measured at 18 hours in the following release test:

### [Release test conditions]

A dissolution test solution is prepared by adding Tween 20 to 1 L of 10 mM PBS buffer (pH 7.4) to 0.05% (w/v), thus preparing a mixed solution, and adding sodium azide and sodium ascorbate to the mixed solution to 0.02% (w/v) and 0.0625% (w/v), respectively.

100 mL of the dissolution test solution is placed in a vial and the microparticles are added thereto in such an amount that the total amount of CBD in the microparticles is 15 mg. After completely sealing the vial, the test is conducted by shaking the vial at 120 rpm while maintaining 50°C. At 18 hours after the start of dissolution, the vial is taken out and left for 3 minutes. Then, 1 mL of the test solution is accurately taken using a syringe, and centrifuged at 3,000 rpm for 3 minutes, and the supernatant is used as a test solution to measure the release of CBD.

A method for preparing a sustained-release injectable composition for treating or preventing a neurological disease according to another embodiment of the present invention may include: a step of preparing an oil phase solution by dissolving cannabidiol (CBD) and a biodegradable polymer in an organic solvent; a step of preparing a water phase solution by dissolving a surfactant in water; and a step of mixing the oil phase solution and the water phase solution to form an emulsion.

In addition, the method may further include: a step of collecting the formed emulsion in the water phase solution to remove residual solvent; a step of washing and freeze-drying the emulsion from which the residual solvent has been removed, thereby producing microparticles; and a step of mixing the freeze-dried microparticles with water for injection.

The oil phase solution and the water phase solution may be injected into the respective microchannels and allowed to flow therethrough, and the emulsion including cannabidiol and the biodegradable polymer may be formed at a point where the flow of the oil phase solution and the flow of the water phase solution intersect each other.

The oil phase solution may further contain butylated hydroxytoluene (BHT).

### Advantageous Effects

The present invention may exhibit the effect of treating or preventing a neurological disease by releasing cannabidiol in a sustained manner for one month or more using microparticles containing cannabidiol.

In addition, the composition of the present invention is a sustained-release injectable composition containing cannabidiol, which is known to have a wide margin of safety upon systemic exposure, and it has fewer side effects caused by long-term use and may exhibit a therapeutic or preventive effect on a neurological disease for one month or more by a single injection, thereby significantly improving the convenience of administration.

### Brief Description of Drawings

FIG. 1 shows the results of a CBD release test for microparticles containing CBD according to one embodiment of the present invention.
FIG. 2 shows the results of a CBD release test for microparticles containing CBD according to one embodiment of the present invention.
FIG. 3 shows the results of a rat pharmacokinetic (pK) test for microparticles according to one embodiment of the present invention.
FIG. 4 shows the test results for changes in body weight of rats after administration of microparticles according to one embodiment of the present invention.
FIG. 5 shows the results of analyzing seizures in a PTZ-induced seizure rat model after administration of microparticles according to one embodiment of the present invention.
FIG. 6 shows the results of analyzing changes in distribution of seizure grades for a PTZ-induced seizure rat model after administration of microparticles according to one embodiment of the present invention.
FIG. 7 shows the result of analyzing seizure-induced mortality for a PTZ-induced seizure rat model after administration of microparticles according to one embodiment of the present invention.
FIG. 8 shows the results of a stability test for microparticles according to one embodiment of the present invention.

### Best Mode

The present invention relates to a sustained-release injectable composition for treating or preventing a neurological disease, including microparticles that release cannabidiol (CBD) in a sustained manner for one month or more, wherein the microparticles include cannabidiol and a biodegradable polymer.

### Mode for Invention

Hereinafter, embodiments of the present invention will be described in detail so that those skilled in the art can easily implement the present invention. However, the present invention may be embodied in various different forms and is not limited to the embodiments described herein.

Cannabidiol (CBD) that is used in the present invention is a physiologically active substance isolated from cannabis. It may be extracted directly from cannabis, or may be extracted CBD, or may be produced by chemical synthesis, and any salt thereof may also be used, without being limited to the above examples.

Cannabis is also called hemp, and was used as cloth in the past. More specifically, hemp fibers are used for fabrics, mosquito nets, ropes, fishing nets, and papermaking materials, and hemp fruits are squeezed to obtain oil, which is used for food, lamp oil, soap, varnish, and paint, and oil cake thereof is used as feed and fertilizer.

The psychoactive chemical delta-9-tetrahydrocannabinol (THC), one of the main components of cannabis, is a component derived from the *Cannabis sativa* or *Cannabis indica* plant, commonly called marijuana, and is classified as an illegal drug in some countries. In countries where it is classified as an illegal drug, storage and handling without special permission are considered as illegal acts. Another representative component is cannabidiol (CBD), which is known to have no psychoactive effects unlike THC. Therefore, various studies have been conducted recently on its use in medicines and cosmetics.

The World Health Organization (WHO) published a report stating that cannabidiol (CBD) oil based on a cannabis extract is effective against epilepsy, Alzheimer's disease (dementia), and the like.

The World Anti-Doping Agency has published the "2018 International Standard for Prohibited Lists" and excluded CBD from the list of prohibited drugs starting this year. CBD is a drug widely used by athletes for pain treatment. Other substances that may be obtained from cannabis, such as hashish and marijuana, have been banned, but CBD oil for medical purposes has been permitted.

As mentioned above, the use of CBD for medical purposes is permitted in various countries.

In 2018, Epidyolex^{®}, an oral solution containing CBD, was approved for the treatment of Lennox-Gastaut syndrome (LGS) and Dravet syndrome (DS), both rare pediatric conditions. While the mechanism of action of CBD has not been clearly understood, CBD is known to reduce neuronal hyperexcitability by regulating intracellular calcium concentrations, and its efficacy has been confirmed in numerous *in vitro* and *in vivo* test systems. It was confirmed that, when Epidiolex^{®} was applied to patient groups in clinical trials, the frequency and severity of seizures were significantly reduced.

However, when CBD is used for medical purposes as described above, it is used in the form of CBD oil. Thus, there is no formulation that increases the convenience of taking CBD, as CBD must be taken in a sustained manner for a long period of time to treat or prevent a specific disease. In addition, the previously approved product is in the form of an oral solution, which has disadvantages in that it difficult to administered the correct dosage and the concentration of the drug in the blood is rapidly reduced.

Accordingly, the present invention provides an injectable composition that may be used for the treatment or prevention of a neurological disease using CBD, and is particularly characterized by including microparticles capable of releasing CBD in a sustained manner for one month or more by a single injection.

The microparticles are characterized by including CBD and a biodegradable polymer.

Accordingly, when the microparticles of the present invention are administered by injection, they may exhibit the effect of administering CBD in a sustained manner by a single administration. Specifically, the microparticles may maintain the blood concentration of CBD at the effective concentration or higher for one month or more by a single administration, may maintain the blood concentration of CBD at the effective concentration or higher for several months by a single administration, may maintain the blood concentration of CBD at the effective concentration or higher for three months by a single administration, may maintain the blood concentration of CBD at the effective concentration or higher for six months by a single administration, and may maintain the blood concentration of CBD at the effective concentration or higher for 12 months by a single administration.

That is, the microparticles may exhibit the effect of releasing CBD in a sustained manner for 1 month or more, 3 months or more, 6 months or more, or 12 months or more.

The effect of releasing CBD in a sustained manner as described above may be affected by the degradation rate of the sustained-release agent, i.e., the biodegradable polymer.

The biodegradable polymer may be selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof.

As an example, the molar ratio of glycolide to lactide in the polylactide-co-glycolide may be about 40:60 to about 90:10, about 40:60 to about 85:15, about 40:60 to about 80:20, about 40:60 to about 75:25, about 45:55 to about 90:10, or about 45:55 to about 80:20, without being limited to the above examples. Preferably, the molar ratio of glycolide to lactide in the polylactide-co-glycolide may be about 75:25 or about 50:50. When a polylactide-co-glycolide having a molar ratio of glycolide to lactide within the above range is used as a sustained-release agent, it may release CBD *in vivo* in a sustained manner for one month or more.

The biodegradable polymer may include one or two or more types of polymer. Specifically, it may include one or more types of polylactide, one or more types of polylactide-co-glycolide, or polylactide and polylactide-co-glycolide. In the present invention, the biodegradable polymer may include, for example, two types of polylactide, a combination of one type of polylactide and one type of polylactide-co-glycolide, two types of polylactide-co-glycolide, three types of polylactides, a combination of two types of polylactide and one type of polylactide-co-glycolide, a combination of one type of polylactide and two types of polylactide-co-glycolide, or the like, and in particular, may include one type of polylactide, one type of polylactide-co-glycolide, or one type of polylactide and one type of polylactide-co-glycolide, without being not limited thereto.

The biodegradable polymer may include polylactide or polylactide-co-glycolide alone or may include one type of polylactide-co-glycolide and one type of polylactide, without being limited to the above examples, and any polymer may be used without limitation as long as it may release CBD *in vivo* in a sustained manner for one month or more.

The microparticles may include CBD and the biodegradable polymer at a weight ratio of 1:1 to 1:10 or 1:2 to 1:9. When the microparticles including CBD and the biodegradable polymer at a weight ratio within the above range are used as an injectable composition, they may be used as a sustained-release injectable composition that releases CBD in a sustained manner within an injectable dose range. That is, if the weight ratio of CBD to the biodegradable polymer is lower than the lower limit of the above range, the amount of the injectable composition will be increased to meet the total dose for administering CBD by injection, which causes a problem of exceeding the amount of the injectable composition that may be administered once to a person. In addition, if the weight ratio of CBD to the biodegradable polymer is higher than the upper limit of the above range, it will not be easy to produce microparticles in which CBD is uniformly distributed.

The neurological disease may be epilepsy. Without being to epilepsy, the microparticles of the present invention may be used for the treatment or alleviation of other neurological diseases, and may be applied without limitation to any disease for which CBD has been proven to be effective in treating or alleviating epilepsy. However, the microparticles may preferably be used for the treatment or alleviation of epilepsy.

The microparticles may release 35% to 80% of the total weight of CBD, as measured at 18 hours in the following release test:

### [Release test conditions]

A dissolution test solution is prepared by adding Tween 20 to 1 L of 10 mM PBS buffer (pH 7.4) to 0.05% (w/v), thus preparing a mixed solution, and adding sodium azide and sodium ascorbate to the mixed solution to 0.02% (w/v) and 0.0625% (w/v), respectively.

100 mL of the dissolution test solution is placed in a vial and the microparticles are added thereto in such an amount that the total amount of CBD in the microparticles is 15 mg. After completely sealing the vial, the test is conducted by shaking the vial at 120 rpm while maintaining 50°C. At 18 hours after the start of dissolution, the vial is taken out and left for 3 minutes. Then, 1 mL of the test solution is accurately taken using a syringe, and centrifuged at 3,000 rpm for 3 minutes, and the supernatant is used as a test solution to measure the release of CBD.

The CBD release test is conducted to confirm the sustained release of CBD using a release test solution. As described above, in order for microparticles containing CBD to release CBD in a sustained manner for one month or more, 35% to 80%, 40% to 80%, or 50% to 80% of the total weight of CBD should be released up to 18 hours in the release test. When CBD is released in an amount within the above range, the microparticles may exhibit the effect of releasing CBD in a sustained manner for one month or more. That is, if CBD is released in an amount lower than the lower limit of the above range, the effect of releasing CBD at the initial stage will be excessively small, so that the effect of treating or alleviating a neurological disease by CBD may not be exhibited, and if CBD is released in an amount higher than the upper limit of the above range, the amount of CBD released will be excessively large, so that the effect of releasing CBD in a sustained manner for one month or more may not be exhibited.

In addition, the microparticles of the present invention may further include butylated hydroxytoluene (BHT).

The CBD may be a compound represented by the following Formula:

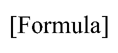

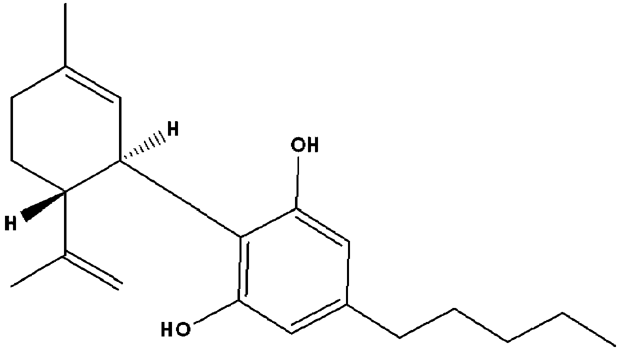

CBD, a compound represented by the above Formula, is a stable compound that is not oxidized upon contact with oxygen in the air. However, if CBD is formulated into microparticles including a biodegradable polymer as in the present invention, there is a problem in that oxidation of CBD occurs.

If CBD is oxidized as described above, the color may change to yellow as described above, and as the structure of the compound changes due to oxidation, a decrease in efficacy may occur.

The butylated hydroxytoluene (BHT) may be contained in the microparticles to prevent the oxidation of CBD. That is, the butylated hydroxytoluene (BHT) may prevent the oxidation of cannabidiol by being oxidized instead of CBD in the microparticles.

The microparticles of the present invention are spherical microparticles as described below, and when the solvent is completely removed through the production process, cannabidiol, the sustained-release agent, and butylated hydroxytoluene (BHT) may be uniformly distributed therein.

At this time, the oxidation reaction of CBD occurs in an environment where the microparticles come into contact with oxygen, but the oxidation reaction of butylated hydroxytoluene (BHT) occurs preferentially to the oxidation reaction of CBD, so that the oxidation reaction of CBD may be prevented.

Although the oxidation reaction of CBD may be prevented by the above-mentioned action, this oxidation-preventing effect is not exhibited by an antioxidant other than butylated hydroxytoluene (BHT), and when the microparticles include butylated hydroxytoluene (BHT), butylated hydroxytoluene (BHT) may be oxidized preferentially to CBD, thereby exhibiting the effect of preventing the oxidation of CBD.

The butylated hydroxytoluene (BHT) may be included in an amount of 0.10 wt% to 1 wt%, 0.10 wt% to 0.50 wt%, 0.11 wt% to 0.40 wt%, or 0.12 wt% to 0.35 wt%, based on the total weight of cannabidiol and the biodegradable polymer. Within the above range, it is possible to produce spherical microparticles, it is possible to maintain an appropriate content range of CBD so that the microparticles may be used as a sustained-release formulation, and it is possible to increase the stability of CBD through the effect of preventing the oxidation of CBD by butylated hydroxytoluene (BHT).

A sustained-release injectable composition including cannabidiol according to another embodiment of the present invention may include: the microparticles containing cannabidiol; and a suspending solvent.

The injectable composition may include a suspending solvent, wherein the suspending solvent includes an isotonic agent, a suspending agent, and a solvent.

More specifically, the isotonic agent may be selected from the group consisting of D-mannitol, maltitol, sorbitol, lactitol, xylitol, sodium chloride, and mixtures thereof, and is preferably D-mannitol, without being limited to the above examples.

The suspending agent is selected from the group consisting of sodium carboxymethylcellulose, polysorbate 80, starch, starch derivatives, polyhydric alcohols, chitosan, chitosan derivatives, cellulose, cellulose derivatives, collagen, gelatin, hyaluronic acid (HA), alginic acid, algin, pectin, carrageenan, chondroitin, chondroitin sulfate, dextran, dextran sulfate, polylysine, titin, fibrin, agarose, fluran, xanthan gum, and mixtures thereof, and is preferably sodium carboxymethylcellulose and polysorbate 80, without being limited to the above examples.

As the solvent, water for injection may be used, and any solvent that may be used as water for injection may be used without limitation.

A method for producing microparticles containing cannabidiol (CBD) according to another embodiment of the present invention may include: a step of preparing an oil phase solution by dissolving CBD and a biodegradable polymer in an organic solvent; a step of preparing a water phase solution by dissolving a surfactant in water; and a step of mixing the oil phase solution and the water phase solution to form an emulsion.

According to the production method, an emulsion may be formed using an oil phase solution, prepared by dissolving CBD, a sustained-release agent, and an antioxidant in an organic solvent, and a water phase solution containing a surfactant.

As described below, according to the present invention, the emulsion is produced by a microfluidic method using a microchannel, without being limited to this production method, and any microparticle production method such as a solvent evaporation method or a membrane method may be applied.

The organic solvent may be one capable of completely dissolving CBD and the biodegradable polymer. Specifically, the organic solvent may be any one or more selected from the group consisting of chloroform, chloroethane, dichloroethane, dichloromethane, trichloroethane, methylene chloride, methanol, and mixtures thereof, and preferably may be selected from the group consisting of methylene chloride, methanol, and mixtures thereof. In addition to the above-listed organic solvents, any organic solvent may be used without limitation, as long as it is capable of completely dissolving CBD and the biodegradable polymer and may be easily selected by those skilled in the art.

The CBD and biodegradable polymer in the organic solvent may be contained at a weight ratio of 1:1 to 1:10, or 1:2 to 1:9.

The biodegradable polymer in the oil phase solution is contained in an amount of 10 to 20 wt%, preferably 12 to 18 wt%, more preferably 15 wt%, without being to the above examples. If the biodegradable polymer is contained in an amount smaller than the lower limit of the above range, it is impossible to prepare an emulsion, and if biodegradable polymer is contained in an amount larger than the upper limit of the above range, there is a problem in that the viscosity of the oil phase solution is excessively high, making it not easy to prepare an emulsion.

The biodegradable polymer is selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof, and is preferably polylactide-co-glycolide (PLGA) and/or polylactide (PLA), without being limited to the above examples.

The oil phase solution may further contain butylated hydroxytoluene (BHT). As described above, the butylated hydroxytoluene (BHT) may be contained in the oil phase solution and may be contained in the microparticles, thereby serving to prevent the oxidation of CBD. The butylated hydroxytoluene (BHT) may be contained in an amount of 0.10 wt% to 1 wt%, 0.10 wt% to 0.50 wt%, 0.11 wt% to 0.40 wt%, or 0.12 wt% to 0.35 wt%, based on the total weight of cannabidiol and the biodegradable polymer.

The water phase solution may contain the surfactant in an amount of 0.1 to 1.0 wt%, 0.2 to 0.5 wt%, or 0.25 wt%, with the remainder being water.

The surfactant may be any one or more selected from the group consisting of methylcellulose, polyvinylpyrrolidone, lecithin, gelatin, polyvinyl alcohol, sorbitan monooleate (e.g., Span^{™} 80, etc.), polyoxyethylene sorbitan fatty acid ester (e.g., Tween^{™} 80, etc.), polyoxyethylene castor oil derivatives, sodium lauryl sulfate, sodium stearate, ester amines, linear diamines, fatty amines, and mixtures thereof, and preferably may be polyvinyl alcohol, without being limited to the above examples, and any surfactant allowing the preparation of perfectly spherical emulsion particles may be used.

After the oil phase solution and the water phase solution are separately prepared as described above, the method of preparing an emulsion using the same is not limited.

However, in the present invention, a method of producing microparticles using a microfluidic method will be described.

A microchip for using the microfluidic method may be formed on a wafer or a glass substrate. Microchannels are formed in the microchip. More specifically, the microchannels include a channel through which the oil phase solution flows, a channel through which the water phase solution flows, and a transport channel. The channel through which the oil phase solution flows and the channel through which the water phase solution flows are formed to meet each other at one point, and one end of the transport channel may be connected to a portion where the two channels are joined.

Injection portions for injecting the oil phase solution and the water phase solution are connected to the channel through which the oil phase solution flows and the channel through which the water phase solution flows, respectively, and a recovery portion for recovering a solution containing an emulsion may be connected to one end of the transport channel.

The microchannels may be formed on a material selected from the group consisting of a glass substrate, a silicon wafer and a polymer film, but the material is not limited to the above examples, and it is possible to use any material on which the microchannels may be formed.

The polymer film may be selected from the group consisting of polyimide, polyethylene, fluorinated ethylene propylene, polypropylene, polyethylene terephthalate, polyethylene naphthalate, polysulfone, and mixtures thereof, without being limited to the above examples.

As an example, aluminum is deposited on a silicon wafer using an e-beam evaporator, and photoresist is patterned on the aluminum using a photolithography technique. Thereafter, the aluminum is etched using the photoresist as a mask, the photoresist is removed, the silicon wafer is etched by deep ion reactive etching (DRIE) using the aluminum as a mask, the aluminum is removed, and then glass is anodically bonded onto the wafer and hermetically sealed, thereby fabricating the microchannels.

The microchannels have an average diameter of 60 to 150 µm, preferably 80 to 100 µm, without being limited to the above example. If microchannels having an average diameter smaller than the lower limit of the above range are used, an emulsion having an excessively small diameter may be produced, which may affect the release and *in vivo* absorption of the effective drug.

In addition, if the microchannels have an average diameter larger than the upper limit of the above range, the average size of the produced microparticles will exceed 120 µm, and foreign body sensation and pain may increase when the microparticles are administered by injection. As the diameter of the microchannels increases, the particle size distribution of the produced microparticles increases, making it difficult to produce microparticles with a uniform particle size.

In addition, the average diameter of the microchannels is closely related not only to the average diameter of the particles, but also to the ratio of the flow rates (µl/min) of the oil phase solution and the water phase solution.

In addition, the cross-sectional width (w) and the cross-sectional height (d) of the microchannel are closely related to the average diameter (d') of the microparticles being produced. The cross-sectional width (w) of the microchannel is in a ratio range of 0.7 to 1.3 with respect to the average diameter (d') of the microparticles, and the cross-sectional height (d) of the microchannel is in a ratio range of 0.7 to 1.3 with respect to the average diameter (d') of the microparticles.

That is, when the average diameter (d') of the microparticles to be produced is determined, the lengths of the cross-sectional width (w) and cross-sectional height (d) of the microchannel should be set in a ratio range of 0.7 to 1.3 with respect to d' so that microparticles having a desired size may be produced.

In order to produce microparticles using the above-described microchip, the oil phase solution may be injected into the channel through which the oil phase solution flows, and the water phase solution may be injected into the channel through which the water phase solution flows, thereby forming an emulsion at the portion where the two channels are joined.

When the oil phase solution and the water phase solution are injected into the respective microchannels, the ratio of the flow rates of the oil phase solution and the water phase solution may be 1:10 to 1:50, 1:15 to 1:40, 1:15 to 1:30, or 1:15 to 1:25. By controlling the ratio of the flow rates of the oil phase solution and the water phase solution as described above, it is possible to produce microparticles having a uniform diameter.

The emulsion formed in the portion where the two channels are joined may be collected using the previously prepared water phase solution, i.e., a mixed solution of the surfactant and water. The prepared water phase solution may be used to form an emulsion by being injected into the microchannel, and may also be used to prevent the aggregation of collected emulsion particles by being placed into a bath.

Residual organic solvent may be removed from the emulsion collected in the bath. The step of removing the residual organic solvent may be performed by stirring the emulsion at a predetermined temperature and a predetermined stirring speed to evaporate and remove the residual organic solvent present in the emulsion. Here, the stirring is performed under the following conditions: a first stirring step at 15 to 20°C for 50 to 70 minutes; a second stirring step at 20 to 40°C for 50 to 70 minutes; and a third stirring step at 40 to 60°C for 1 to 3 hours.

The stirring speed is the same for all of the first to third stirring steps, and the stirring speed may be 300 to 500 rpm, or 400 rpm.

As described above, the stirring temperature is gradually increased as the stirring process progresses. By increasing the temperature stepwise, it is possible to control the evaporation rate of the organic solvent present in the emulsion.

The temperature at which the oil phase solution and the water phase solution flow through the microchannels is also 5 to 20°C, preferably 10°C. That is, after the solutions flow along the microchannels and intersect each other to produce an emulsion, the collected emulsion is maintained at a constant low temperature of 5 to 20°C until it is stirred in the first stirring step. Only when the emulsion preparation process is maintained at a low temperature, it is possible to produce and maintain spherical particles. That is, if a low-temperature condition is not used, a problem arises in that it is difficult to produce particles having a uniform spherical shape.

Thereafter, in the second stirring step, the temperature is increased gradually and the stirring time is increased so that the organic solvent present in the emulsion may be moved slowly to the surface and evaporated from the surface, thereby minimizing the effect of the evaporation of the organic solvent on the appearance of the emulsion. That is, if the organic solvent is rapidly evaporated, a problem may arise in that the surfaces of the finally produced microparticles are not smooth and pores are formed, due to the evaporation of the organic solvent. In order to prevent this problem, the evaporation rate of the organic solvent may be controlled by increasing the temperature gradually as described above and also increasing the stirring process time, and due to this control of the evaporation rate of the organic solvent, it is possible to control the surface appearance of the produced microparticles.

In the third stirring process, after the organic solvent in the emulsion is extracted with the external water phase, the external water phase is stirred at an increased temperature near the boiling point of the organic solvent, thereby removing the saturated organic solvent from the water phase, thus facilitating the removal of residual organic solvent from the emulsion.

Lastly, a step of washing and drying the microparticles from which residual organic solvent has been removed is performed. In this step, the microparticles from which the organic solvent on the surface has been completely removed by stirring are washed several times with sterile filtered purified water to remove the surfactant remaining in the microparticles, and then freeze-dried.

The finally produced microparticles are in a form in which CBD is uniformly distributed in the microparticles made of the spherical biodegradable polymer, and may include CBD and the biodegradable polymer at a weight ratio of 1:1 to 1:10, or 1:2 to 1:9.

In addition, as described above, the finally produced microparticles have butylated hydroxytoluene (BHT) uniformly distributed therein in addition to CBD, so that when they come into contact with oxygen in the air, butylated hydroxytoluene (BHT) may be preferentially oxidized, thereby preventing the oxidation of CBD.

The weight ratio between CBD and the biodegradable polymer contained in the microparticles is the same as the weight ratio in the oil phase solution used to prepare the emulsion. Specifically, as the solutions pass through the microchannels to form the emulsion and the organic solvent is completely removed from the emulsion, the produced microparticles may include CBD and the biodegradable polymers at the same weight ratio as the weight ratio in the oil phase solution.

The microparticles may have an average diameter of 30 to 70 µm, 30 to 65 µm, or 30 to 60 µm. In addition, the standard deviation for the average diameter may be 1 to 30 µm, 1 to 20 µm, 1 to 10 µm, or 1 to 7 µm. It can be confirmed that it is possible to produce uniform particles within the above diameter range. When the uniform particles are used as a sustained-release injectable composition, they may increase the convenience of administration by reducing foreign body sensation, prevent initial burst release upon *in vivo* injection, and exhibit the effect of releasing CBD in a sustained manner.

### Production Example

### Production of Microparticles

### 1) Preparation of Oil Phase Solution

An oil phase solution was prepared by dissolving cannabidiol, a biodegradable polymer consisting of a lactide-glycolide copolymer and a lactide copolymer alone or in combination, and butylated hydroxytoluene (BHT) in methylene chloride. Here, the content of the biodegradable polymer in the oil phase solution was 15 wt%, and the weight ratio between cannabidiol and the biodegradable polymer was 1:1 to 1:10.

### 2) Preparation of Water Phase Solution

A water phase solution was prepared by dissolving polyvinyl alcohol in water. The content of polyvinyl alcohol in the water phase solution was 0.25 wt%.

### 3) Production of Microparticles

The oil phase solution and the water phase solution were injected into microchannels formed on a silicon wafer, thereby producing microparticles. Here, the ratio of the flow rate of the oil phase solution to the flow rate of the water phase solution was 1:20, and the temperature was maintained at 10.0°C. The produced microparticles were collected in a bath containing the water phase solution, followed by stirring at a speed of 400 rpm at 10.0°C for 1 hour, 30.0°C for 1 hour, and 45.0°C for 2 hours.

### 4) Washing and Collection

After completion of the stirring, the microparticles were washed with sterile filtered purified water, freeze-dried, and then collected in powder form.

Microspheres were produced using the components and contents shown in Table 1 below according to the method described above.

**[Table 1]**

| Purpose of mixing | Component name | Comp. Example 1 | Comp. Example 2 | Example 1 | Example 2 | Example 3 | Example 4 | Example 5 | Example 6 | Example 7 |
|---|---|---|---|---|---|---|---|---|---|---|
| | | | | | | Quantity (mg) | | | | |
| API:POLYMER ratio | | 1:1 | 1:10 | 1:2 | 1:3 | 1:4 | 1:4 | 1:9 | 1:4 | 1:4 |
| Active ingredient | Cannabidiol | 100.00 | | | | | | | | |
| Sustained-release agent | Lactide-glycolide copolymer (50:50) | - | - | - | - | 400.00 | - | - | 267.00 | - |
| | Lactide-glycolide copolymer (75:25) | 100.00 | 1,000.00 | 200.00 | 300.00 | - | 400.00 | 900.00 | 133.00 | 267.00 |
| | Lactide copolymer | - | - | - | - | - | - | - | - | 133.00 |
| Antioxidant | Butylated hydroxytoluene | 1.00 | | | | | | | | |
| Organic solvent | Methylene chloride | 666.67 | 6,666.67 | 1,333.33 | 2,000.00 | 2,666.67 | 2,666.67 | 6,000.00 | 2,666.67 | 2,666.67 |
| Total mass (mg) | | 201.00 | 1,101.00 | 301.00 | 401.00 | 501.00 | 501.00 | 1,001.00 | 500.00 | 501.00 |

### Experimental Example 1

### CBD Release Test

### 1) Preparation of Standard Solution

About 15 mg of cannabidiol standard was taken and placed in a 100-mL flask. About 50 mL of diluting solution (acetonitrile) was added thereto and the mixture was sonicated for 10 minutes. After cooling sufficiently, the test solution was added up to the marked line, filtered through a 0.45 µm filter (water-soluble PTFE), and then used as a standard solution (concentration: 0.15 mg/mL).

### 2) Preparation of Dissolution Test Solution

A dissolution test solution was prepared by adding Tween 20 to 1 L of 10 mM PBS buffer (pH 7.4) to 0.05% (w/v), and then adding sodium azide and sodium ascorbate to the mixture to 0.02% (w/v) and 0.0625% (w/v), respectively.

### 3) Preparation of Test Solution

Each sample was precisely weighed to be 15 mg as the main ingredient, and placed in a vial, and 100 mL of the dissolution test solution was accurately added thereto. After sealing the vial, the test was conducted by shaking the vial at a speed of 120 rpm and at a constant temperature of around 50°C. The vial was placed horizontally. The vial containing the sample was taken out at 2, 18, and 72 hours from the start of dissolution and left for 3 minutes so as to allow the sample in the test solution to settle. Next, 1 mL of the test solution was accurately taken using a syringe and centrifuged at 3,000 rpm for 3 minutes, and the supernatant was used as a test solution.

### 4) HPLC conditions

- Detector: UV (220 nm)
- Column: ODS column (4.6*150 mm, 5 µm)
- Flow rate: 1.0 mL/min
- Mobile phase: methanol: purified water = 85:15 (isocratic method)
- Calculation formula: Main peak area of the test solution*amount of standard taken*purity of standard (%)*dilution ratio/peak area of main component of standard solution*equivalent amount (mg) of cannabidiol in test solution

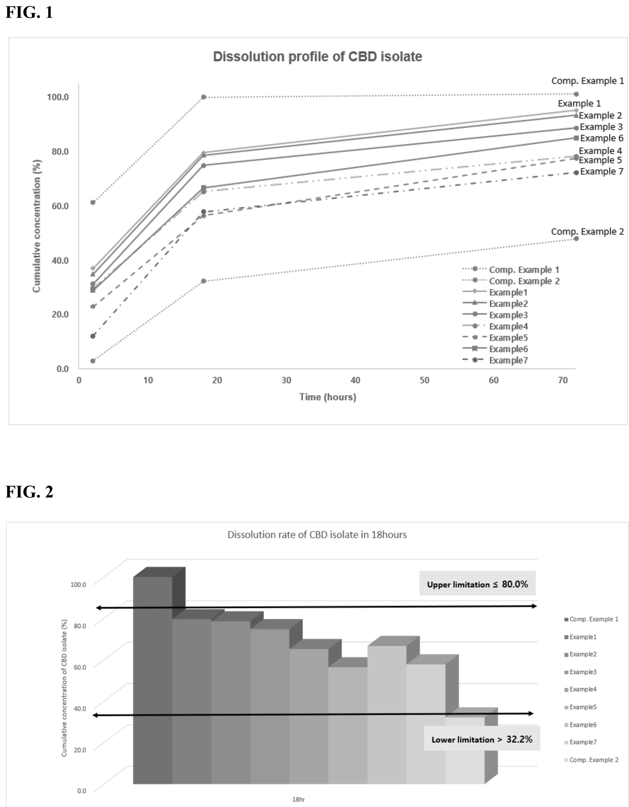

The results of conducting the CBD release test under the above-described experimental conditions are shown in FIGS. 1 and 2 and Table 2 below.

**[Table 2]**

| | 2 hours | 18 hours | 72 hours |
|---|---|---|---|
| Comparative Example 1 | 61.2±1.1 | 100.0±2.2 | 101.2±0.5 |
| Comparative Example 2 | 3.0±3.9 | 32.2±0.1 | 47.9±0.1 |
| Example 1 | 36.9±0.1 | 79.6±7.4 | 95.2±0.1 |
| Example 2 | 34.8±0.9 | 78.6±2.4 | 93.3±2.8 |
| Example 3 | 31.2±6.5 | 74.8±0.1 | 88.6±0.5 |
| Example 4 | 29.6±0.4 | 65.2±3.8 | 78.2±0.7 |
| Example 5 | 22.9±10.6 | 56.4±2.5 | 77.4±1.8 |
| Example 6 | 28.9±0.6 | 66.7±1.2 | 85.0±0.3 |
| Example 7 | 12.1±8.2 | 57.8±1.0 | 72.3±0.2 |

Referring to the above experimental results, it can be confirmed that Comparative Example 1 showed CBD release amounts of 61.2% at 2 hours, 100% at 18 hours, and 101.2% (almost completely released) at 72 hours. Comparative Example 1 has a problem in that it cannot exhibit the effect of releasing CBD in a sustained manner for a long period of time, as the initial release amount of CBD is large.

In addition, Comparative Example 2 showed a CBD release amount of 47.9% at 72 hours, indicating that it can exhibit the effect of releasing CBD in a sustained manner for a long period of time. However, it showed a CBD release amount of only 3% at 2 hours, indicating that the initial CBD release amount was excessively small. Thus, it has a problem in that the effect of CBD on the treatment and alleviation of neurological diseases is insufficient.

On the other hand, Examples 1 to 7 could show a CBD release amount of 50% to 80% at 18 hours, and did not show complete release of CBD even at 72 hours, suggesting that they exhibited a drug release effect in a sustained manner for a long period of time.

### Experimental Example 2

### Rat Pharmacokinetic Test

The experimental conditions for conducting the rat pharmacokinetic test are as follows:

**[Table 3]**

| | |
|---|---|
| Administration route | Subcutaneous (SC) injection |
| Administration frequency and duration | Single administration |
| Administration site and dose (mg/kg) | Dorsal skin tissue; IVL5005: 9 mg/head (administered SC) |
| Method of administration | SC: Inject subcutaneously into dorsal skin tissue using a 1-ml disposable syringe. Administer once at the start of treatment. |

As experimental animals, 6-week-old male SD rats were purchased from Coretech, acclimated for a week, and then tested. The experimental diet was a general feed for experimental animals (Orient), and the rats were raised with free access to water and feed.

### General Symptoms

During the experimental period, changes in general symptoms were observed twice (morning and afternoon) a day for all animals, and body weight was measured upon introduction and division into groups.

### Blood Sampling

At a total of 14 time points, including time 0 (before SC administration of the test substance to the acclimated rats) and 4 hr, 8 hr, 24 hr, 3 d, 5 d, 1 w, 2 w, 4 w, 6 w, 8 w, and 12 w after SC administration of the test substance, 500 to 1,000 µL of blood was sampled at each time point, and then immediately, the blood was centrifuged at 1,000 g for 10 minutes to separate the serum, which was then frozen and stored at -70°C.

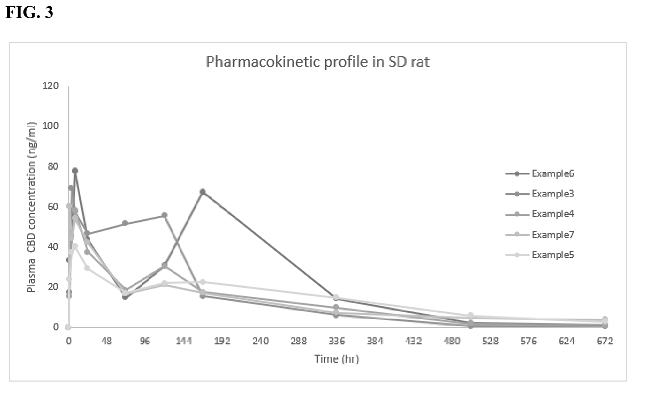

The results of measuring the blood drug concentration after administration of the microparticles of Examples 3 to 3 in the above experiment are shown in Table 4 below and FIG. 3.

**Table 4]**

| Rat PK | | | Example 3 | Example 4 | Example 5 | Example 6 | Example 7 |
|---|---|---|---|---|---|---|---|
| h | d | w | | | | | |
| 0 | 0 | - | 0 | 0 | 0 | 0 | 0 |
| 1 | 0.04 | - | 17.2714 | 15.4024 | 23.873 | 33.4146 | 60.258 |
| 4 | 0.17 | - | 69.4666 | 45.7472 | 37.2256 | 44.7864 | 44.4638 |
| 8 | 0.33 | - | 57.6326 | 58.4982 | 40.104 | 77.6844 | 54.142 |
| 24 | 1 | - | 46.793 | 37.4204 | 29.2382 | 43.8268 | 42.2964 |
| 72 | 3 | - | 51.7345 | 18.31025 | 16.805 | 14.789 | 16.7002 |
| 120 | 5 | - | 55.662 | 30.336 | 22.031 | 30.6045 | 21.1844 |
| 168 | 7 | 1 | 15.3444 | 17.496 | 22.3244 | 67.6294 | 17.2954 |
| 336 | 14 | 2 | 6.0052 | 9.634 | 14.6402 | 14.133 | 7.107 |
| 504 | 21 | 3 | 0.3138 | 1.6684 | 5.6058 | 1.9916 | 4.5834 |
| 672 | 28 | 4 | 0.0356 | 0.5952 | 2.5394 | 0.7818 | 3.7714 |
| 1176 | 49 | 7 | 0 | 0.3644 | 0.7612 | 0 | 3.4008 |

| | | | | | | | |
|---|---|---|---|---|---|---|---|
| (unit: µg/mL) | | | | | | | |

Referring to the above experimental results, it can be confirmed that Examples 3 to 7 of the present invention maintained the blood CBD concentration for at least 1 month. That is, it was confirmed that, at 4 weeks, Example 3 showed 0.0356 µg/mL Example 4 showed 0.5952 µg/mL, Example 5 showed 2.5394 µg/mL, Example 6 showed 0.7818 µg/mL, and Example 7 showed 3.7714 µg/mL.

In addition, it can be confirmed that Examples 4, 5 and 7 exhibited the effect of releasing CBD in a sustained manner as they showed the blood CBD concentration even at 7 weeks.

### Experimental Example 3

Induction of Epilepsy by Pentylenetetrazole (PTZ) and Administration of Test Substance

In order to evaluate the seizure-suppressing effect of administration of the microparticles of the present invention in an epilepsy-induced model, administration groups were set up as follows.

**[Table 5]**

| Group | Administration route | Number of animals | Dose (mg/head) | Dose (ml/head) |
|---|---|---|---|---|
| G1. Vehicle control | SC | 10 | - | 2 |
| G2. CBD solution | IP | 10 | 60 | 2 |
| G3. IVL5005-30 | SC | 10 | 30 | 2 |
| G4. IVL5005-60 | SC | 10 | 60 | 2 |

Administration to rats was performed in the manner shown in Table 6 below.

**[Table 6]**

| | |
|---|---|
| Administration routes of the test substances | IVL5005: Subcutaneous (SC) injection |
| | CBD solution: Intraperitoneal (IP) injection |
| Administration frequency and duration | IVL 5005: administered once (D1); CBD solution: administered twice (D1 and D14) |
| Administration site and dose (mg/kg) | IVL5005: dorsal skin tissue, 30 (G3) to 60 (G4) mg/head (administered SC); CBD solution: dorsal abdominal area, 60 mg/head (administered IP) |
| Method of administration | SC: Inject subcutaneously into dorsal skin tissue using a 1-ml disposable syringe. |
| | Administer once at the start of treatment. IP: Inject intraperitoneally using a 1-ml disposable syringe. Administer at the start of treatment (D1) and on week 2 (D14). |

G3 and G4 were administered to rats at different doses using the above-described Example 7.

After the completion of acclimatization, the rats were divided into groups. On day 1 (D1), the test substances G1 to G4 were administered intraperitoneally and subcutaneously, and 30 minutes later, PTZ (pentylenetetrazole, Sigma, USA) dissolved in 0.9% sterile saline was administered intraperitoneally at a dose of 37.5 mg/kg to induce epileptic seizures. In the normal group, only the same amount of saline was administered. Then, PTZ was repeatedly administered at time points D2, D3, D5, D7, D14, D21, and D28 to induce epileptic seizures. The intensity of the seizures was graded and evaluated for 30 minutes immediately after PTZ administration at each time point according to the following criteria:

**[Table 7]**

| Seizure stage | Characteristics (Muhammad, 2009) |
|---|---|
| 0 | No response |
| 1 | Ear and facial twitching |
| 2 | Convulsive waves axially through the body |
| 3 | Myoclonic jerks and rearing |
| 4 | Clonic convulsions with the animal falling on its side |
| 5 | Repeated severe tonic-clonic convulsions or lethal convulsions |
| 6 | Death associated with seizure |

### Test Results

### Body Weight Change

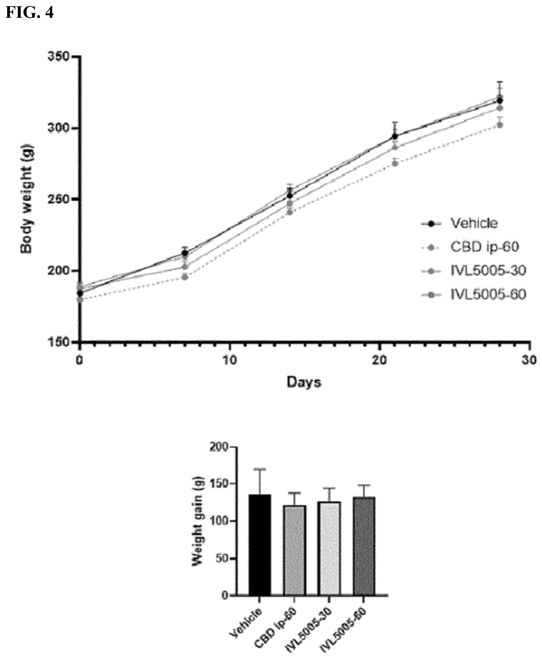

The test results are shown in FIG. 4. During the test period, body weight continued to increase in all the groups without decrease. Although the weight gain in the group administered CBD intraperitoneally was slightly smaller than that in the other groups, no statistically significant difference was observed in any group.

### Seizure Analysis

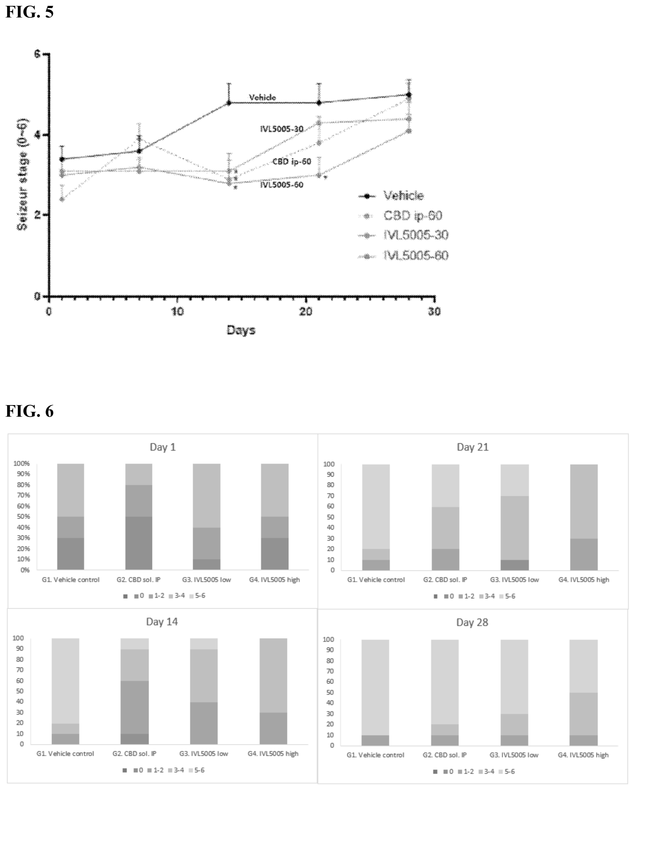

The results of quantitative analysis of the intensity of PTZ-induced seizures classified into stages 0 to 6 are shown in FIG. 5. As shown in FIG. 5, in the vehicle-administered group, seizure of grade 3.4 was induced on day 1, and then the degree of seizure gradually increased, and seizure of grade 5 was maintained for the following 2 to 4 weeks.

In the group (G2) administered the CBD solution intraperitoneally, the seizure intensity was grade 2.4 on day 1, which was lower than that in the vehicle control group, but increased to grade 3.9 in week 1, which was not different from that in the vehicle control group. However, after additional CBD administration in week 2, the seizure intensity decreased again to grade 2.9, but increased again to grade 4.9 in weeks 3 and 4, indicating a loss of the seizure-suppressing effect.

In the IVL5005-administered groups (G3 and G4), the intensity of seizures did not increase in both dose groups from day 1 to week 2 and remained lower than that in the vehicle control group. In particular, in week 2, when the intensity of seizures in the vehicle control group began to increase significantly, the intensity of seizures in both dose groups was observed to be statistically significantly lower than that in the vehicle control group. In weeks 3 and 4, the intensity of seizures in the 30 mg/head group (G3) increased to grade 4.4, but was still lower than that in the vehicle control group. In the 60 mg/head group (G4), the intensity of seizures was suppressed without increasing until week 3, and then increased to grade 4.1 in week 4.

### Changes in Distribution of Seizure Grades

FIG. 6 shows the results of analyzing the percent (%) distribution of animals corresponding to each grade at each time point at which seizure intensity was measured. On day 1, no severe seizures of grade 5 or higher were induced in all the groups, and in week 1, severe seizures of grade 5 or higher were observed in 1 to 3 animals in each group, but no individuals of grade 5 or higher were observed in the groups administered IVL5005 (G3 and G4). In the vehicle control group, 80% to 90% of individuals showed severe seizures of grade 5 or higher during a period from week 2 to week 4. In the group (G2) administered CBD intraperitoneally, severe seizures were observed in only 10% of individuals at the time of additional administration in week 2, but the number of individuals with grade 5 or higher gradually increased in weeks 3 and 4, and more than half of individuals showed grade-5 seizures in week 4. In the IVL5005-administered groups (G3 and G4), seizures of grade 5 or higher were observed in less than 30% even until 2 to 3 weeks, and in particular, in the 60 mg/head group (G4), seizures of grade 5 or higher were not observed until week 3, and seizures of grade 5 were observed in 50% even in week 4, showing the lowest frequency among all the groups.

### Analysis of Seizure-Induced Mortality

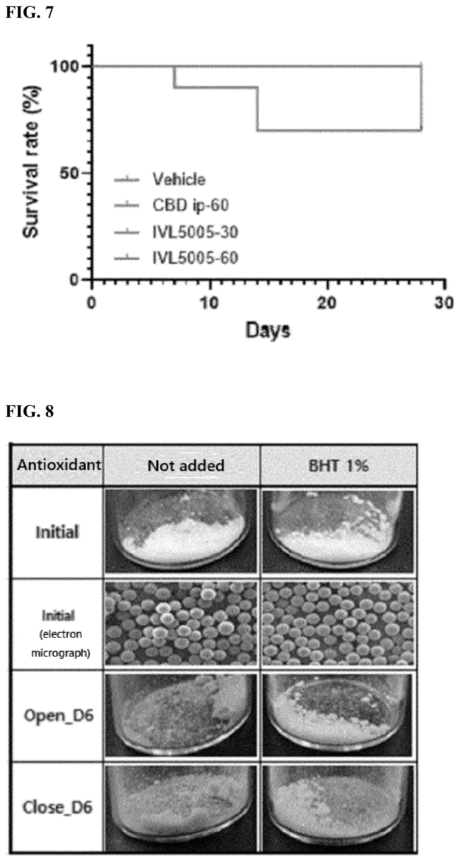

FIG. 7 shows the results of analyzing seizure-induced mortality. In individuals with severe seizures, a large amount of secretions were produced from the nasal and oral cavities, respiratory distress occurred, and death occurred in some cases. As a result of analyzing the mortality rate in each group, death occurred earliest in the vehicle control group (G1) from week 1, and two additional animals died in week 2. In the group (G2) administered CBD intraperitoneally, no deaths occurred until week 3, but three animals died in week 4. In the IVL5005-administered groups (G3 and G4), no deaths occurred until the end of the test.

### Experimental Example 4

### Evaluation of Stability

Microparticles were produced using the same composition as that of Example 1 in the same manner as in Example 1, except that BHT was not included.

About 100 mg of the microparticles were weighed into a 5-mL glass vial which was then stored in a capped or uncapped (close/open) stability chamber. Stability evaluation was performed under long-term conditions (temperature: 25±2°C, relative humidity: 60±5%), and daily appearance changes were observed to check yellowing. Meanwhile, the initial sample and the sample on day 6 after storage were tested for their encapsulation efficiency to check for any changes in content.

The experimental results are shown in FIG. 8.

It was confirmed that there was no change in color immediately after producing the microparticles. In addition, as a result of checking the presence of yellowing on day 6, it was confirmed that the color changed to yellow in the case in which BHT was not included, regardless of whether the glass vial was capped.

On the other hand, it was confirmed that, in the case in which BHT was included in an amount of 1 wt% as in Example 1, the color did not change, indicating excellent stability.

Although the preferred embodiments of the present invention have been described in detail above, the scope of the present invention is not limited thereto, and various modifications and improvements made by those skilled in the art using the basic concept of the present invention as defined in the appended claims also fall within the scope of the present invention.

### Industrial Applicability

The present invention relates to a sustained-release injectable composition for treating or preventing a neurological disease and a method for producing the same.

## Claims

1. A sustained-release injectable composition for treating or preventing a neurological disease, comprising microparticles that release cannabidiol (CBD) in a sustained manner for one month or more,
wherein the microparticles comprise cannabidiol and a biodegradable polymer.

2. The sustained-release injectable composition of claim 1, wherein the microparticles comprise cannabidiol and the biodegradable polymer at a weight ratio of 1:1 to 1:10.

3. The sustained-release injectable composition of claim 1, wherein the biodegradable polymer is selected from the group consisting of polylactic acid, polylactide, polylactic-co-glycolic acid, polylactide-co-glycolide (PLGA), polyphosphazine, polyiminocarbonate, polyphosphoester, polyanhydride, polyorthoester, polycaprolactone, polyhydroxyvalerate, polyhydroxybutyrate, polyamino acids, and combinations thereof.

4. The sustained-release injectable composition of claim 3, wherein the polylactide-co-glycolide has a monomer ratio of lactide: glycolide of 40:60 to 90:10.

5. The sustained-release injectable composition of claim 1, wherein the neurological disease is epilepsy.

6. The sustained-release injectable composition of claim 1, wherein the microparticles release 35% to 80% of the total weight of CBD, as measured at 18 hours in the following release test:
[Release test conditions]
A dissolution test solution is prepared by adding Tween 20 to 1 L of 10 mM PBS buffer (pH 7.4) to 0.05% (w/v), thus preparing a mixed solution, and adding sodium azide and sodium ascorbate to the mixed solution to 0.02% (w/v) and 0.0625% (w/v), respectively.
100 mL of the dissolution test solution is placed in a vial and the microparticles are added thereto in such an amount that the total amount of CBD in the microparticles is 15 mg. After completely sealing the vial, the test is conducted by shaking the vial at 120 rpm while maintaining 50°C. At 18 hours after the start of dissolution, the vial is taken out and left for 3 minutes. Then, 1 mL of the test solution is accurately taken using a syringe, and centrifuged at 3,000 rpm for 3 minutes, and the supernatant is used as a test solution to measure the release of CBD.

7. A method for preparing a sustained-release injectable composition for treating or preventing a neurological disease, comprising:
a step of preparing an oil phase solution by dissolving cannabidiol (CBD) and a biodegradable polymer in an organic solvent;
a step of preparing a water phase solution by dissolving a surfactant in water; and
a step of mixing the oil phase solution and the water phase solution to form an emulsion.

8. The method of claim 7, further comprising:
a step of collecting the formed emulsion in the water phase solution to remove residual solvent;
a step of washing and freeze-drying the emulsion from which the residual solvent has been removed, thereby producing microparticles; and
a step of mixing the freeze-dried microparticles with water for injection.

9. The method of claim 7, wherein the oil phase solution and the water phase solution are injected into the respective microchannels and allowed to flow therethrough, and the emulsion containing cannabidiol and the biodegradable polymer is formed at a point where the flow of the oil phase solution and the flow of the water phase solution intersect each other.

10. The method of claim 7, wherein the oil phase solution further contains butylated hydroxytoluene (BHT).
